Europäisches Patentamt

European Patent Office

Office européen des brevets

(19)

(11) Publication number: **0 189 079**
**B1**

# EUROPEAN PATENT SPECIFICATION

(12)

(45) Date of publication of patent specification: **17.08.88**

(21) Application number: **86100325.9**

(22) Date of filing: **12.01.86**

(51) Int. Cl.⁴: **C 07 C 2/84,** C 07 C 9/06,
C 07 C 11/04 // B01J23/10

(54) Process for the production of hydrocarbons.

(30) Priority: **14.01.85 JP 3164/85**

(43) Date of publication of application:
**30.07.86 Bulletin 86/31**

(45) Publication of the grant of the patent:
**17.08.88 Bulletin 88/33**

(84) Designated Contracting States:
**AT BE CH DE FR GB IT LI LU NL SE**

(56) References cited:
**DE-A-3 237 079**
**DE-A-3 248 432**
**GB-A-2 148 935**
**US-A-4 499 323**
**US-A-4 499 324**

**CHEMIKER-ZEITUNG, vol. 107, 1983,
Heidelberg (DE); W.HINSEN et al.: "Oxidative
Kupplung von Methan zu C2-
Kohlenwasserstoffen in Gegenwart
unterschiedlicher Katalysatoren", pp. 223-226**

(73) Proprietor: **IDEMITSU KOSAN COMPANY
LIMITED**
No. 1-1, 3-chome, Marunouchi Chiyoda-ku
Tokyo (JP)

(72) Inventor: **Otsuka, Kiyoshi**
No. 3-2-1231, 2-chome Minamisuna Kotoh-ku
Tokyo (JP)
Inventor: **Morikawa, Akira**
24-11, 2-chome, Fujigaoka Midori-ku
Yokohama-shi Kanagawa-ken (JP)

(74) Representative: **Türk, Dietmar, Dr. rer. nat. et al
Türk, Gille + Hrabal Patentanwälte Bruckner
Strasse 20
D-4000 Düsseldorf 13 (DE)**

Courier Press, Leamington Spa, England.

# 0 189 079

**Description**

## BACKGROUND OF THE INVENTION

The present invention relates to a process for the production of hydrocarbons. More particularly, it is concerned with a process for producing industrially useful hydrocarbons having two or more carbon atoms, such as ethane and ethylene, from methane with high efficiency by the use of specific catalysts.

Methane contained in natural gas, for example, has been used to produce industrially useful hydrocarbons such as ethane, ethylene and acetylene. Various methods of production of such useful hydrocarbons are known. In any of the known methods, the hydrocarbons are produced by the oxidative dehydrogenation reaction at temperatures as high as more than 1,000 degree centrigrade (°C). In industrial applicaton, therefore, the methods have disadvantages in that expensive heat-resistant equipment and a large amount of energy are needed and in that the operation is difficult. In recent years, several methods to produce hydrocarbons having two or more carbon atoms from methane at lower temperature have been proposed. These methods, however, are unsuitable for practical use because the conversion of methane and selectivities for ethane and ethylene, for example, are low.

## SUMMARY OF THE INVENTION

As a result of extensive investigations to produce useful hydrocarbons such as ethane and ethylene from methane with high efficiency at relatively low temperatures, it has been found that the object can be attained by using specific catalysts containing rare earth metals.

The present invention relates to a process for producing hydrocarbons having two or more carbon atoms from methane which comprises contacting methane with a catalyst containing at least one rare earth metal.

## BRIEF DESCRIPTION OF THE DRAWINGS

Fig. 1 is a graph showing the effect of reaction temperature on selectivity and conversion when samarium oxide was used as a catalyst; and

Fig. 2 is a graph showing the effect of reaction temperature on selectivity and conversion when dysprosium oxide was used as a catalyst.

## DETAILED DESCRIPTION OF THE INVENTION

As the methane that is used as a feedstock in the present invention, as well as high purity methane gas, natural gas composed of various kinds of gas can be used. In use the methane feedstock may be diluted with inert gas such as helium, nitrogen and argon, or steam.

In the process of the present invention, it is necessary for the reaction to be carried out in the presence of oxygen. The concentration of oxygen in the reaction system can be determined taking into consideration a degree of reaction and an explosion limit. As the oxygen to be supplied to the reaction system, as well as pure oxygen, mixed gas containing oxygen and other gas not exerting adverse influences on the reaction (e.g., air) can be used.

The catalysts of the present invention contain at least one rare earth metal. The rare earth metal includes scandium (Sc), yttrium (Y), lanthanum (La), cerium (Ce), praseodymium (Pr), neodymium (Nd), promethium (Pm), samarium (Sm), europium (Eu), gadolinium (Gd), terbium (Tb), dysposium (Dy), holmium (Ho), erbium (Er), thulium (Tm), itterbium (Yb) and lutetium (Lu). Of these metals, Sc, Y, La, Nb, Sm, Eu, Gd, Dy, Ho, Er, Tm, Yb and Lu are particularly suitable.

Examples of the caalyst of the present invention are one metal selected from the above metals, a mixture of two or more metals selected from the above metals, the oxide of one metal selected from the above metals, and a mixture of the oxides of two or more metals selected from the above metals. In particular, metal oxides are suitable for use in the present invention. Representative examples of such metal oxides are scandium oxide ($Sc_2O_3$), yttrium oxide ($Y_2O_3$), lanthanum oxide ($La_2O_3$), samarium oxide ($Sm_2O_3$), europium oxide ($Eu_2O_3$), gadolinium oxide ($GD_2O_3$), disprosium oxide ($Dy_2O_3$), holmium oxide ($Ho_2O_3$), erbium oxide ($Er_2O_3$), thulium oxide ($Tm_2O_3$), itterbium oxide ($Yb_2O_3$), and lutetium oxide ($Lu_2O_3$).

In the process of the present invention, the metal or metal oxide as the catalyst can be used as such. In practical use, however, since the surface area of the catalyst is desirable to be large, it is suitable for the catalyst to be used in a powdery or granular form.

The metal or metal oxide may be deposited on an inorganic oxide carrier. Innorganic oxide carriers which can be used for this purpose include silica, alumina, magnesia, titania, and zirconia. Deposition of the metal or metal oxide on such inorganic carrier can be carried out by known techniques such as impregnation and kneading.

To the catalyst of the present invention can be added the oxides of alkali metals and/or alkaline earth metals. Representative examples of such oxides are calcium oxide (CaO), magnesium oxide (MgO), sodium oxide ($Na_2O$), and potassium oxide ($K_2O$). Addition of such oxides retards complete oxidation and increases the selectivity of methane to hydrocarbons having two or more carbon atoms.

In accordance with the process of the present invention, the methane feedstock is contacted with the above-prepared catalyst in the presence of oxygen. The reaction is continuously carried out by passing methane and oxygen, if necessary, in combination with inert gas as a diluent through a tube-shaped flow

2

reactor packed with the catalyst. Thus, in the process of the present invention, the desired hydrocarbons are produced with high efficiency. In connection with reaction conditions, the pressure is atmospheric pressure to 50 kg/cm²G (49 bar) and preferably atmospheric pressure to 10 kg/cm²G (9,8 bar) and the temperature is 500 to 1,000°C and preferably 650 to 750°C.

In the process of the present invention, various hydrocarbons having two or more carbon atoms are produced, and in particular, ethane and ethylene are obtained in a selectivity as high as about 90%.

As described above, the process of the present invention permits to continuously and efficiently produce hydrocarbons having two or more carbon atoms, particularly ethylene and ethane useful as starting materials for production of various chemical products from methane in a very high selectivity under relatively low temperature conditions. Accordingly the process of the present invention is very useful from an industrial standpoint.

The present invention is described below in greater detail with reference to the following examples.

### Example 1

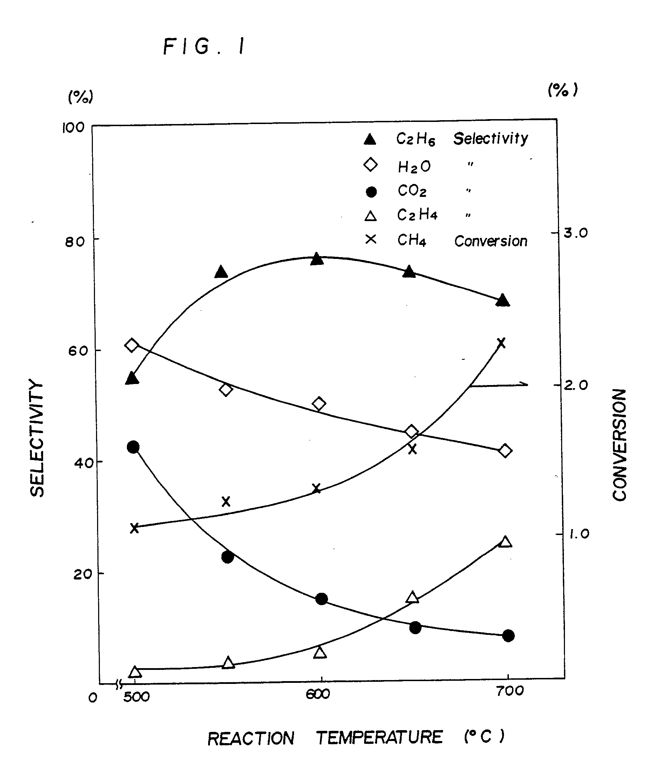

A quartz glass reactor was charged with 0.5 gram (g) of samarium oxide ($Sm_2O_3$), and 4.5 milliliters per minute (ml/min) of methane, 0.1 ml/min of oxygen and 20.4 ml/min of helium were passed through the reactor while heating in an electric furnace. The heating temperature was adjusted to 500°C, 550°C, 600°C, 650°C or 700°C. Reaction products obtained at each heating temperature were analysized by gas chromatography. This gas chromatographic analysis showed that the reaction products were ethylene, ethane, carbon dioxide and water. The results are shown in Fig. 1. The selectivity in Fig. 1 is calculated based on a carbon atom.

### Example 2

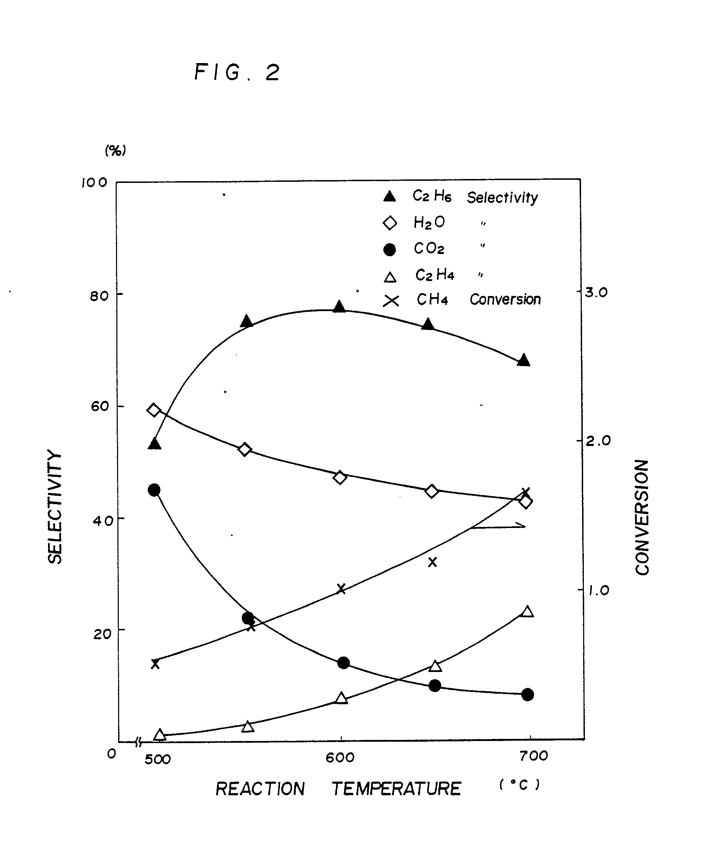

The procedure of Example 1 was repeated wherein 0.5 g of dysprosium oxide was used as the catalyst. The results are shown in Fig. 2.

### Examples 3 to 13

A predetermined amount of a catalyst as shown in Table 1 was placed in a quartz glass reactor, and 4.5 ml/min of methane, 0.1 ml/min of oxygen and 20.4 ml/min of helium were passed through the reactor while heating at 700°C in an electric furnace.

Selectivities and rates of formation of reaction products, ethylene, ethane, carbon dioxide and water are shown in Table 1.

## Table 1

| Run No. | Catalyst Type | Catalyst Amount (g) | Selectivity (%)[1] Ethane | Selectivity (%)[1] Ethylene | Selectivity (%)[1] $CO_2$ | Rate of Formation ($\mu mol/m^2 \cdot min$)[2] Ethane | Rate of Formation ($\mu mol/m^2 \cdot min$)[2] Ethylene | Rate of Formation ($\mu mol/m^2 \cdot min$)[2] $CO_2$ | Rate of Formation ($\mu mol/m^2 \cdot min$)[2] Water |
|---|---|---|---|---|---|---|---|---|---|
| Example 3 | $Sc_2O_3$ | 0.5 | 57 | 18 | 25 | – | – | – | – |
| " 4 | $Y_2O_3$ | " | 54 | 23 | 23 | 0.8 | 0.3 | 0.7 | 2.4 |
| " 5 | $La_2O_3$ | " | 57 | 29 | 14 | 0.45 | 0.2 | 0.2 | 1.1 |
| " 6 | $Nd_2O_3$ | " | 61 | 26 | 13 | 0.55 | 0.2 | 0.2 | 1.4 |
| " 7 | $Eu_2O_3$ | " | 55 | 29 | 16 | 0.4 | 0.25 | 0.25 | 1.3 |
| " 8 | $Gd_2O_3$ | " | 58 | 26 | 16 | 1.4 | 0.65 | 0.8 | 3.7 |
| " 9 | $Ho_2O_3$ | " | 72 | 16 | 12 | 2.1 | 0.4 | 0.7 | 4.5 |
| " 10 | $Er_2O_3$ | " | 72 | 11 | 17 | 1.2 | 0.2 | 0.6 | 2.7 |
| " 11 | $Tm_2O_3$ | " | 74 | 15 | 11 | 0.9 | 0.2 | 0.3 | 1.8 |
| " 12 | $Yb_2O_3$ | " | 70 | 16 | 14 | 1.0 | 0.2 | 0.4 | 2.3 |
| " 13 | $Lu_2O_3$ | " | 50 | 30 | 20 | 0.4 | 0.2 | 0.3 | 1.3 |

Note:   *1 Calculated based on a carbon atom.

*2 Amount ($\mu mol$) formed per square meter of the catalyst surface area and per minute.

0 189 079

## Examples 14 to 17

A quartz glass reactor was charged with 1.0 g of samarium oxide ($Sm_2O_3$), and a mixed gas of methane, oxygen and helium was passed through the reactor at a rate of 25 ml/min in such a manner that the methane partial pressure and oxygen partial pressure were maintained at predetermined values, and the total pressure was 100 kilo Pascal (KPa), while heating at 700°C in an electric furnace. Reaction products obtained were analyzed by gas chromatography.

The results are shown in Table 2.

## Example 18

The procedure of Example 14 was repeated wherein the heating temperature was changed to 720°C.
The results are shown in Table 2.

## Examples 19 to 21

A quartz glass reactor was charged with 2.22 g of lithium-containing samarium oxide ($Li/Sm_2O_3$) (lithium content: 7 percent by weight (wt%)), and a mixed gas of methane, oxygen and helium was passed through the reactor at a rate of 25 ml/min in such a manner that the methane partial pressure and oxygen partial pressure were maintained at predetermined values and the total pressure was 100 KPa, while heating at 700°C in an electric furnace. Reaction products obtained were analyzed by gas chromatography.

The results are shown in Table 3.

## Example 22

The procedure of Example 19 was repeated wherein the heating temperature was changed to 720°C.
The results are shown in Table 3.

Table 2

| Run No. | | Methane Partial Pressure (KPa) | Oxygen Partial Pressure (KPa) | Conversion (%) | | CO + $CO_2$ | Selectivity (%) [1] | | |
| --- | --- | --- | --- | --- | --- | --- | --- | --- | --- |
| | | | | Methane | Oxygen | | Ethylene | Ethane | Ethylene + Ethane |
| Example | 14 | 17.5 | 3.39 | 15.7 | 89.6 | 48.9 | 27.4 | 23.7 | 51.1 |
| " | 15 | 17.9 | 4.20 | 18.4 | 90.7 | 51.4 | 27.1 | 21.5 | 48.6 |
| " | 16 | 19.2 | 7.94 | 27.4 | 92.6 | 62.7 | 22.8 | 14.5 | 37.3 |
| " | 17 | 8.9 | 4.20 | 31.0 | 90.5 | 63.9 | 21.7 | 14.4 | 36.1 |
| " | 18 | 8.7 | 5.00 | 36.4 | 92.5 | 67.7 | 20.6 | 11.7 | 32.3 |

Note: *1 Calculated based on a carbon atom.

Table 3

| Run No. | | Methane Partial Pressure (KPa) | Oxygen Partial Pressure (KPa) | Conversion (%) | | Selectivity (%)[1] | | | Ethylene + Ethane |
|---|---|---|---|---|---|---|---|---|---|
| | | | | Methane | Oxygen | $CO + CO_2$ | Ethylene | Ethane | |
| Example | 19 | 19.3 | 5.23 | 18.6 | 71.7 | 41.1 | 34.7 | 24.2 | 58.9 |
| " | 20 | 17.9 | 8.10 | 25.3 | 67.3 | 50.7 | 31.3 | 18.0 | 49.3 |
| " | 21 | 8.2 | 4.77 | 30.4 | 64.9 | 53.2 | 28.9 | 17.9 | 46.8 |
| " | 22 | 8.3 | 5.09 | 35.1 | 75.1 | 57.0 | 28.0 | 15.0 | 43.0 |

Note: *1 Calculated based on a carbon atom.

## Claims

1. A process for producing hydrocarbons having two or more carbon atoms from methane which comprises contacting the methane with a catalyst containing at least one rare earth metal in the presence of oxygen.

2. The process as claimed in Claim 1, wherein the hydrocarbons having two or more carbon atoms are ethylene, ethane or a mixture thereof.

3. The process as claimed in Claim 1, wherein the rare earth metal is scandium, yttrium, lanthanum, neodynium, samarium, europium, gadolinium, dysprosium, holmium, erbium, thulium, ytterbium or lutetium.

4. The process as claimed in Claim 1, wherein the catalyst is the oxide of a rare earth metal.

## Patentansprüche

1. Ein Verfahren zur Herstellung von Kohlenwasserstoffen mit zwei oder mehr Kohlenstoffatomen aus Methan, welches den Kontakt des Methans mit einem Katalysator, der wenigstens ein seltenes Erdmetall enthält, in Gegenwart von Sauerstoff umfaßt.

2. Das Verfahren nach Anspruch 1, worin die Kohlenwasserstoffe mit zwei oder mehr Kohlenstoffatomen Ethylen, Ethan oder ein Gemisch von beiden sind.

3. Das Verfahren nach Anspruch 1, worin das seltene Erdmetall Scandium, Yttrium, Lanthan, Neodym, Samarium, Europium, Gadolinium, Dysprosium, Holmium, Erbium, Thulium, Ytterbium oder Lutetium ist.

4. Das Verfahren nach Anspruch 1, worin der Katalysator ein Oxid eines seltenen Erdmetall ist.

## Revendications

1. Procédé pour la production d'hydrocarbures ayant deux ou plus de deux atomes de carbone à partir du méthane, qui comprend la mise en contact du méthane avec un catalyseur contenant au moins un métal des terres rares en présence d'oxygène.

2. Procédé selon la revendication 1, dans lequel les hydrocarbures ayant deux ou plus de deux atomes de carbone sont l'éthylène, l'éthane ou un de leurs mélanges.

3. Procédé selon la revendication 1, dans lequel le métal des terres rares est le scandium, l'yttrium, le lanthane, le néodyme, le samarium, l'europium, le gadolinium, le dysprosium, l'holmium, l'erbium, le thulium, l'ytterbium ou le lutéium.

4. Procédé selon la revendication 1, dans lequel le catalyseur est l'oxyde d'un métal des terres rares.

# F I G . 1

FIG. 2

(%)

SELECTIVITY

CONVERSION

▲ $C_2H_6$  Selectivity
◇ $H_2O$  "
● $CO_2$  "
△ $C_2H_4$  "
✕ $CH_4$  Conversion

REACTION  TEMPERATURE  (°C)